# EUROPÄISCHE PATENTANMELDUNG

(11) **EP 3 409 266 A1**
(43) Veröffentlichungstag der Anmeldung: **05.12.2018**
(21) Anmeldenummer: 18176961.3
(22) Anmeldetag: 29.11.2011
(51) Int. Cl.: A61K 8/60, A61Q 5/02, A61Q 5/12, A61Q 19/00, A61Q 19/10

(54) **WIRKSTOFFKOMBINATIONEN AUS GLUCOSYLGLYCERIDEN UND EINEM ODER MEHREREN MILDEN TENSIDEN**

(30) Priorität: 23.12.2010 DE 102010055767
(62) Teilanmeldung aus: 11790950.7
(71) Anmelder: Beiersdorf AG, 20253 Hamburg (DE)
(72) Erfinder: Kröpke, Rainer, 22869 Schenefeld (DE); Frese, Christian, 20259 Hamburg (DE); Scherner, Cathrin, 22851 Norderstedt (DE); Neufhoff, Henrike, 22359 Hamburg (DE); Hentrey, Stefanie, 22848 Norderstedt (DE)

(57) **Zusammenfassung**

Wirkstoffkombinationen aus
(i) sauren Konservierungsmitteln und
(ii) einem oder mehreren Glucosylglyceriden.

## Beschreibung

Die vorliegende Erfindung betrifft Wirkstoffkombinationen aus Glucosylglyceriden und einem oder mehreren milden Tensiden sowie deren Verwendung auf dem Gebiete der kosmetischen sowie der pharmazeutischen Dermatologie.

Insbesondere betrifft die vorliegende Erfindung Wirkstoffe und kosmetische oder dermatologische Zubereitungen, solche Wirkstoffkombinationen enthaltend.

Bevorzugt betrifft die vorliegende Erfindung waschaktive kosmetische Zubereitungen.

Die äußerste Schicht der Epidermis, das Stratum corneum (Hornschicht), ist als wichtige Barriereschicht von besonderer Bedeutung u.a. für den Schutz vor Umwelteinflüssen und Austrocknung. Die Hornschicht wird im Kontakt mit der Umwelt ständig abgenutzt und muss deshalb ununterbrochen erneuert werden.

Ein heute in der Fachwelt weitverbreitetes Hautmodell fasst das Stratum corneum als ZweiKomponenten-System, ähnlich einer Ziegelsteinmauer (Ziegelstein-Mörtel-Modell), auf. In diesem Modell entsprechen die Korneozyten (Hornzellen) den Ziegelsteinen, die kompliziert zusammengesetzte Lipidmembran in den Interzellularräumen entspricht dem Mörtel.

Außer ihrer Barrierewirkung gegen externe chemische und physikalische Einflüsse tragen die epidermalen Lipide auch zum Zusammenhalt der Hornschicht bei und haben Einfluss auf die Hautglätte. Im Gegensatz zu den Talgdrüsenlipiden, die keinen geschlossenen Film auf der Haut ausbilden, sind die epidermalen Lipide über die gesamte Hornschicht verteilt.

Das äußerst komplexe Zusammenwirken der feuchtigkeitsbindenden Substanzen und der Lipide der oberen Hautschichten ist für die Regulation der Hautfeuchte sehr wichtig. Daher enthalten Kosmetika in der Regel, neben ausgewogenen Lipidabmischungen und Wasser, wasserbindende Substanzen. Hierzu zählen u.a. Polyole wie Glycerin, Sorbit und Xylit, ethoxylierte Polyole sowie hydrolysierte Proteine. Weitere Anwendung finden die im natürlichen Feuchthaltefaktor (sogenannter Natural Moisturizing Factor = NMF) enthaltenen Substanzen, wie z.B. Harnstoff, Kohlenhydrate (z. B. Glucose) und Aminosäuren (z. B. Serin). Diese Substanzen sind daher für die Pflegeleistung eines kosmetischen Produktes von besonderer Bedeutung, insbesondere auch aufgrund ihrer relativ guten Haut- und Schleimhautverträglichkeit.

Der Wunsch nach sauberer Haut ist wohl so alt wie die Menschheit, denn Schmutz, Schweiß und Reste abgestorbener Hautpartikel bieten den idealen Nährboden für Krankheitserreger und Parasiten aller Art. Die Lust an der Körperhygiene wurde stetig verstärkt, als in den 60er Jahren des 20. Jahrhunderts neben der "klassischen" Seife auch flüssige Reinigungsmittel mit neu entwickelten synthetischen Tensiden formuliert werden konnten. Baden und Duschen sind seitdem aus unserem täglichen Leben nicht mehr wegzudenken. Den Verbrauchern stehen heutzutage eine Vielzahl von Produkten für die Reinigung der verschiedenen Körperpartien zur Verfügung.

Reinigung bedeutet das Entfernen von (Umwelt-) Schmutz und bewirkt damit eine Erhöhung des psychischen und physischen Wohlbefindens. Die Reinigung der Oberfläche von Haut und Haaren ist ein sehr komplexer, von vielen Parametern abhängiger Vorgang. Zum einen sollen von außen kommende Substanzen wie beispielsweise Kohlenwasserstoffe oder anorganische Pigmente aus unterschiedlichsten Umfeldern sowie Rückstände von Kosmetika oder auch unerwünschte Mikroorganismen möglichst vollständig entfernt werden. Zum anderen sind körpereigene Ausscheidungen wie Schweiß, Sebum, Haut- und Haarschuppen ohne tiefgreifende Eingriffe in das physiologische Gleichgewicht abzuwaschen.

Kosmetische Reinigungszubereitungen für Haut und Haare, die beispielsweise als Shampoos, Duschbäder und flüssige Seifen angeboten werden, müssen nicht nur ein gutes Reinigungsvermögen aufweisen, sondern sollen weiterhin gut verträglich sein und einen für Haut und Haare pflegenden Effekt aufweisen. Auch bei einer häufigen Anwendung soll es dabei nicht zu einer Entfettung oder Trockenheit der Haut oder der Haare kommen.

Kosmetische Reinigungszubereitungen, die besonders schonend zu Haut und Haar sind, enthalten in der Regel besonders milde Tenside, wie z.B. Zuckertenside oder Aminosäuretenside. Während diese Tenside eine schonende Reinigung der Haut und Haare ermöglichen, ist das Schaumvermögen dieser Tenside nicht geeignet, die Anforderungen der Verbraucher an den Schaum eines Reinigungsprodukts zu erfüllen. Der Schaum sollte dabei besonders ergiebig, cremig und reichhaltig sein. Weiterhin sind die sensorischen Eigenschaften von Reinigungszubereitungen enthaltend milde Tenside nicht zufrieden stellend.

Auf der anderen Seite enthalten kosmetische Reinigungszubereitungen, die einen besonders cremigen und reichhaltigen Schaum erzeugen, oft hohe Mengen an anionischem Sulfattensid, welches dafür bekannt ist, besonders aggressiv zur Haut zu sein und eine Austrocknung der Haut zu verursachen, die sich oft durch gerötete, rissige und spannende Haut offenbart.

Es besteht daher der Bedarf an Zubereitungen, die die Nachteile des Standes der Technik beheben.

Es war nach all diesem überraschend und nicht vorhersehbar, dass Wirkstoffkombinationen aus
(i) einem oder mehreren milden Tensiden
   Und
(ii) einem oder mehreren Glucosylglyceriden
   bzw. kosmetische Zubereitungen, solche Wirkstoffkombinationen enthaltend, die Nachteile des Standes der Technik beseitigen.

Es wurde überraschend festgestellt, dass Kombinationen aus milden Tensiden mit Glucosylglyceriden die Nachteile des Standes der Technik beheben können. Diese Zubereitungen zeichnen sich durch eine sehr gute Hautverträglichkeit und hervorragende sensorische Eigenschaften im Vergleich zu Zubereitungen des Standes der Technik aus. Ferner haben diese Zubereitungen sehr gute Schaumeigenschaften.

Die Milde eines Tensides oder Tensidsystems lässt sich durch die Bestimmung der Quotienten aus Hämolysewert und Denaturierungsindex charakterisieren. Der Quotient L/D repräsentiert das Verhältnis von Hämolysewert (L) zu Denaturierungsindex (D) und wird durch den Standard RBC-Test bestimmt.

Der Standard RBC-Test dient zur Abschätzung von in-vivo Augenschleimhautreizpotentialen von Tensiden und tensidhaltigen Produkten. Das Verfahren beruht auf der Tatsache, dass Tenside stark mit Zellmembranen und Proteinen interagieren. Beide Effekte werden photometrisch durch Analyse des natürlichen Blutfarbstoffs Oxyhämoglobin (HbOa) bestimmt. Im Gegensatz zu anderen zellbasierten Systemen ist der RBC-Test in der Lage zwischen Schädigungen der Zellmembran (Hämolyse) und Proteindenaturierung (Denaturierungsindex) zu differenzieren.

### Hämolyse

Ein definiertes Aliquot isolierter Kalbserythrozyten wird mit einer Reihe steigender Konzentrationen des zu untersuchenden Musters (Stammlösung für Rohstoffe 0,1 Gew.-% Aktivgehalt der waschaktiven Substanz in PBS; Stammlösung für Fertigprodukte 1:100 w/v in PBS) für 10 Minuten unter Schütteln bei RT inkubiert. PBS ist ein üblicher Standard-PhosphatPuffer (pH 7,4) mit folgender Zusammensetzung:

| | |
|---|---|
| Na₂HPO₄ •2 H₂O | 3,95 g |
| KH₂PO₄ | 0,76 g |
| NaCl | 7,20 g |
| Glucose•1 H₂O | 1,80 g |
| Aqua dest. oder "highly purified water" | ad 1000 ml |

Die Inkubationsperiode wird durch schnelle Hochgeschwindigkeitszentrifugation beendet. Nach Zentrifugation werden die gewonnenen Überstände photometrisch auf ihren Gehalt an freigesetztem Hämoglobin (HbO2) bei 530 nm analysiert. Daraus wird der relative Hämolysegrad berechnet und die Konzentrations-Response-Kurve mit dem H50-Wert [µl/ml] als Kenngröße bestimmt. Dieser gibt die Konzentration des Prüfmusters an, bei der 50% des Hämoglobins freigesetzt werden. Je höher der Wert, desto verträglicher ist das untersuchte Muster.

### HbO₂-Denaturierung

Ein definiertes Aliquot isolierter Kalbserythrozyten wird mit einer fixen Konzentration des Prüfmusters (Konzentration für Rohstoffe 0,1 Gew.-% Aktivgehalt der waschaktiven Substanz in PBS; Konzentration für Fertigprodukte 1% w/v in PBS) für 10 Minuten unter Schütteln bei RT inkubiert und dann zentrifugiert. Die Änderung der spektralen Absorption bei 575 nm und 540 nm wird im Vergleich zum nativen HbO2 gemessen. Aus dem Verhältnis der Absorptionswerte zueinander wird der Denaturierungsindex DI [%] berechnet. Als 100%-Standard dient Natriumlaurylsulfat (0,1% Aktivgehalt).

Je geringer der Wert, desto verträglicher ist das untersuchte Muster.

### L/D-Quotient

Der Quotient (L/D-Wert) stellt das Verhältnis der Kenngrößen von Hämolyse (H50) und Denaturierung (DI) dar und wird zur Charakterisierung und Klassifizierung der untersuchten Prüfmuster verwendet.

Tenside oder Tensidmischungen werden erfindungsgemäß als mild angesehen, wenn der L/D-Wert mindestens 1 beträgt. Zum Vergleich dazu hat das häufig in Reinigungsprodukten eingesetzte Natriumlaurylethersulfat einen L/D-Wert von ca. 0,3 und wird als reizend klassifiziert. Sofern in einer Tensidmischung Natriumlaurylethersulfat eingesetzt wird, hat die resultierende Tensidmischung erfindungsgemäß einen L/D-Wert von mindestens 1.

Bevorzugte milde Tenside sind
- Alkylpolyglucoside wie z.B. Laurylglucosid, Decylglucosid und Cocoglucosid,
- Amphoacetate wie z.B. Natriumcocoamphoacetat, Dinatriumcocoamphodiacetat und Natriumlauroamphoacetat,
- Betaintenside wie z.B. Cocamidopropylbetain oder Cocobetain,
- Aminosäuretenside wie z.B. Natriumlauroylglutamat und Dinatriumcocoylglutamat,
- Ethoxylierte Glyceride wie z.B. PEG-7 Glycerylcocoat,
- Taurate wie z.B. Natriummethylcocoyltaurat,
- Sarkosinate wie z.B. Natriumlauroylsarcosinat
- Sulfosuccinate wie z.B. Dinatrium PEG-5 Laurylcitratsulfosuccinat

In der folgenden Tabelle sind bevorzugte milde Tenside mit ihren L/D-Werten aufgeführt:

| **Rohstoff** | **H50** | **DI** | **L/D** |
|---|---|---|---|
| Cocamidopropylbetain | 56,1 | 2,6 | 21,65 |
| Dinatriumcocoylglutamat | 136,6 | 77,9 | 1,75 |
| Dinatrium PEG-5 Laurylcitratsulfosuccinat | 645 | 5,5 | 117 |
| Natriumlauroylsarcosinat | 458 | 58,4 | 7,84 |
| Dinatriumcocoamphodiacetat | 36,4 | 1,5 | 24 |
| Natriumcocoamphoacetat | 26 | 1 | 26 |
| Laurylglucosid | 84,1 | 3,2 | 20,4 |
| Decylglucosid | 127,7 | 3,9 | 33,4 |
| Dinatriumlaurylethersulfosuccinat | 167,7 | 40,4 | 4,15 |
| Natriumlauroylglutamat | 312,3 | 10,5 | 29,9 |
| PEG-7 Glycerylcocoat | 115,2 | 4,9 | 23,5 |

Besonders bevorzugte milde Tenside sind Alkylpolyglucoside, wie z.B. Laurylglucosid und Decylglucosid, Betaintenside, wie z.B. Cocamidopropylbetain oder Cocobetain, Aminosäuretenside, wie z.B. Natriumlauroylglutamat und Dinatriumcocoylglutamat und Ethoxylierte Glyceride wie z.B. PEG-7 Glycerylcocoat.

Bevorzugte Konzentration der milden Tenside ist 0,01 bis 20%, besonders bevorzugt 0,1 - 15 %, besonders bevorzugt 1 % - 10%.

Vorteilhaft im Sinne der vorliegenden Erfindung sind Glucosylglyceride der allgemeinen Formel 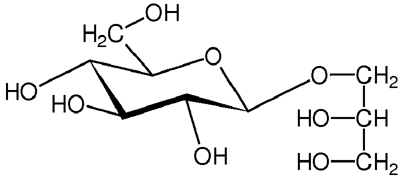 und/oder der allgemeinen Formel 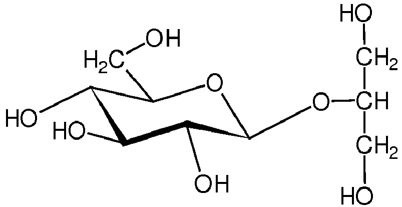 und/oder der allgemeinen Formel 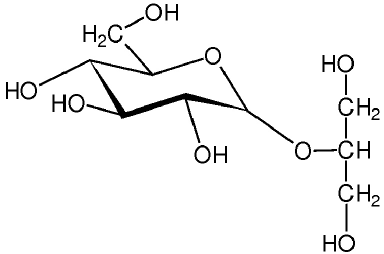 und/oder der allgemeinen Formel 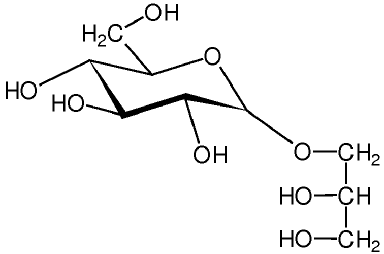 werden erfindungsgemäß bevorzugt.

Erfindungsgemäß sind solche Zubereitungen besonders vorteilhaft, die dadurch gekennzeichnet sind, dass das oder die Glucosylglycerid in der Wasser- und/oder Ölphase in Konzentrationen von 0,001 - 40,00 Gew.-%, bevorzugt 0,005 - 15,00 Gew.-%, besonders bevorzugt 0,01 - 12,00 Gew.-%, jeweils bezogen auf das Gesamtgewicht der Zusammensetzung, vorliegen.

Es ist erfindungsgemäß vorteilhaft, das molare Verhältnis von einem oder mehreren Glucosylglyceriden zu einem oder mehreren milden Tensiden aus dem Bereich von 100 : 1 bis 1 : 1000 , bevorzugt 50 : 1 bis 1 : 500, insbesondere bevorzugt 20 : 1 bis 1 : 100 zu wählen.

Erfindungsgemäß vorteilhaft ist die Verwendung der erfindungsgemäßen Zubereitung als kosmetische Reinigungszubereitung. Dabei wird die erfindungsgemäße Zubereitung bevorzugt als Duschgel, Schaum- und Wannenbad, Shampoo und/oder Gesichtsreiniger verwendet.

Es ist erfindungsgemäß vorteilhaft, wenn die erfindungsgemäße kosmetische Zubereitung in einer Flasche, Quetschflasche, Pumpspray-, oder Aerosoldose aufbewahrt und aus dieser heraus angewendet werden. Entsprechend sind auch Flaschen, Quetschflaschen, Doppelkammerpackmittel, Pumpspray-, oder Aerosoldosen, welche eine erfindungsgemäße Zubereitung enthalten, erfindungsgemäß.

Die erfindungsgemäße Zubereitung enthält vorteilhaft weitere Tenside. Diese liegen erfindungsgemäß vorteilhaft in einer Konzentration von 0,1 bis 5 Gewichts-%, und erfindungsgemäß bevorzugt in einer Konzentration von 1 bis 3 Gewichts-%, jeweils bezogen auf das Gesamtgewicht der Zubereitung, in der Zubereitung vor. Erfindungsgemäß besonders bevorzugt ist dabei der Einsatz von Nariumlaurylethersulfat oder Natriummyristylethersulfat als weiterem Tensid.

Es ist ebenfalls vorteilhaft, den Zubereitungen im Sinne der vorliegenden Erfindung kationische Polymere zuzufügen. Geeignete kationische Polymere sind beispielsweise
- quaternisierte Cellulose-Derivate, wie z.B. Polyquaternium-10, wie sie unter den Bezeichnungen Celquat und Polymer JR im Handel erhältlich sind
- kationische Guar-Derivate, wie insbesondere die unter den Handelsnamen Cosmedia Guar und Jaguar vertriebenen Produkte
- polymere Dimethyldiallylammoniumsalze und deren Copolymere mit Estern und Amiden von Acrylsäure und Methacrylsäure, wie insbesondere die unter den Bezeichnungen Merquat 100 und Merquat 550 im Handel erhältlichen Produkte.

Vorteilhaft im Sinne der vorliegenden Erfindung ist es insbesondere, kationisches Polymer oder Mischungen aus kationischen Polymeren in einer Konzentration von 0.01 bis 2 Gewichts-%, bevorzugt in einer Konzentration von 0.05 bis 1.5 Gewichts-% und besonders bevorzugt von 0.1 bis 1.0 Gewichts-%, jeweils bezogen auf das Gesamtgewicht der Zubereitung, einzusetzen.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Die erfindungsgemäß vorteilhaften einsetzbaren Wirk-, Hilfs- und Zusatzstoffe sind dabei keineswegs auf die hier namentlich erwähnten Stoffe und Verbindungen beschränkt.

Erfindungsgemäß besonders vorteilhafte Wirkstoffe sind insbesondere Niacinamid, Panthenol, Polidocanol, [gamma]-Oryzanol, Ubichinone (insbesondere Q-10) Kreatin, Kreatinin, Biotin (Vitamin H), Vitamin E und Vitamin E-acetat, Pflanzenextrakte, wie z.B. Bambusextrakt, Wasserlilienextrakt, den alpha-Hydroxysäuren, wie z.B. Zitronensäure, Weinsäure, Apfelsäure, Salzen, wie z.B. Calciumsalzen oder Meeresmineralien, BHT, Propylgallat sowie UV-Filter (z.B. besonders vorteilhaft Benzophenon-4).

Derartige Wirkstoffe können erfindungsgemäß vorteilhaft in Konzentrationen (Einzelkonzentration eines Wirkstoffes) von 0,001 bis 5 Gewichts-%, jeweils bezogen auf das Gesamtgewicht der Zubereitung, in den Zubereitungen enthalten sein.

Es ist ferner erfindungsgemäß von Vorteil, wenn den erfindungsgemäßen Zubereitungen Effektstoffe (z.B. Farb- und/oder Wirkstoffkügelchen, Glitterstoffe u.a.) zugefügt werden und/oder die Zubereitung mit stabilen Luftblasen und -bläschen versehen wird.

Erfindungsgemäß vorteilhafte Trübungsmittel/Perlglanzmittel bzw. Mischungen sind unter anderem:
- PEG-3 Distearat (z.B. CUTINA TS der Firma Cognis),
- eine Kombination aus Glycoldistearat, Glycerin, Laureth-4 und Cocamidopropylbetain (z.B. Euperlan PK 3000 und Euperlan PK 4000 der Firma Cognis),
- eine Kombination aus Glycoldistearat, Cocosglucosiden, Glyceryloleat und Glycerylstearat (z.B. Lamesoft TM Benz der Firma Cognis).
- Styrol/Acrylat Copolymere (z.B. Acusol OP 301 von Rohm & Haas)

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wässrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten.

Erfindungsgemäß vorteilhafte Ausführungsformen der vorliegenden Erfindung sind dadurch gekennzeichnet, dass die Zubereitung ebenfalls Polyacrylate als Verdickungsmittel enthält.

Erfindungsgemäß vorteilhafte Polyacrylate sind Polymere der Acrylsäure, insbesondere solche, die aus der Gruppe der sogenannten Carbomere oder Carbopole (Carbopol<(R)> ist eigentlich eine eingetragene Marke der B. F. Goodrich Company) gewählt werden. Vorteilhafte Carbopole sind beispielsweise die Typen 907, 910, 934, 940, 941, 951, 954, 980, 981, 1342, 1382, 2984 und 5984 oder auch die Typen ETD (Easy-to-disperse) 2001, 2020, 2050, Aqua-SF1 wobei diese Verbindungen einzeln oder in beliebigen Kombinationen untereinander vorliegen können.

Ferner vorteilhaft im Sinne der vorliegenden Erfindung sind die den Acrylat-Alkylacrylat-Copolymeren vergleichbaren Copolymere aus C10-30-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die unter den Handelsbezeichnungen Pemulen TR1 und Pemulen TR2 bei der B. F. Goodrich Company erhältlichen.

Es ist erfindungsgemäß von besonderem Vorteil, wenn als Polyacrylate C10 bis C30-Alkylacrylat Copolymere eingesetzt werden.

Das Verdickungsmittel ist in dem Gel z.B. in einer Menge zwischen 0,1 und 30 Gew.-%, bevorzugt zwischen 0,5 und 15 Gew.-%, enthalten.

Es folgen vorteilhafte Ausführungsbeispiele der vorliegenden Erfindung.

**verwendete Rohstoffe**

| **Handelsname** | **Bezeichnung** | **Aktivgehalt** | **Hersteller** |
|---|---|---|---|
| Atlas G-4280 | PEG-80 Sorbitanlaurat | ∼75% | Croda |
| Carbopol Aqua SF-1 | Acrylatcopolmyer | ∼30% | Lubrizol |
| Carbopol 980 | Carbomer | 100% | Lubrizol |
| Cetiol HE | PEG-7 Glycerylcocoat | 100% | Cognis |
| Cosmospheres BMM-M | Mannitol + Microkristalline Cellulose + Milchsäure + Cl 74160 | 73,75% 25% + 1% + Cl 74160 | Pelletech |
| Cosmospheres WTS-M | Lactose + mikrokristalline Cellulose | 75% + 25% | Pelletech |
| Eumulgin HRE 40 | PEG-40 Hydriertes Rizinusöl | 100% | Cognis |
| Euperlan PK 771 | Natriumlaurethsulfat + Glycoldistearat + Laureth-10 | 25% + 15% + 5% | Cognis |
| Euperlan PK 900 | PEG-3 Distearat + Natriumlaurethsulfat | 25% + 14% | Cognis |
| Glucamate DOE-120 | PEG-120 Methylglucosedioleat | 100% | Lubrizol |
| Hostapon CT Paste | Natriummethylcocoyltaurat | ∼30% | Clariant |
| Inducos NO. 13/3 | Polyethylen | 100% | Induchem |
| Inducos 14/1 | Polyethylen | 100% | Induchem |
| Keltrol CG-RD | Xanthangummi | 100% | Cp Kelco |
| Lamesoft PO 65 | Coco Glucosid + Glyceryloleat | 40% + 30% | Cognis |
| Merquat 550 | Polyquaternium-7 | ∼8% | Nalco |
| Opulyn 301 | Styrol/Acrylatcopolymer | ∼40% | Rohm & Haas |
| Oxetal VD 92 | PEG-90 Glycerylisostearat + Laureth-2 | 70% + 7% | Zschimmer & Schwarz |
| Plantacare 1200 UP | Laurylglucosid | ∼50% | Cognis |
| Plantacare 2000 UP | Decylglucosid | ∼50% | Cognis |
| Polyox WSR-301 | PEG-90M | ∼95% | Dow Chemical |
| Purasal S/PF 50 | Natriumlactat | ∼50% | Purac |
| Rewoderm LI 520-70 | PEG-200 Hydriertes Glycerylpalmat | ∼70% | Evonik Goldschmidt |
| Rewopol SB CS 50 | Dinatrium PEG-5 Laurylcitrat Sulfosuccinat | ∼30% | Evonik Goldschmidt |
| Rewoteric AM C | Natriumcocoamphoacetat | ∼90% | Evonik Goldschmidt |
| Tego Betain F 50 | Cocamidopropylbetain + Glycerin | ∼36% + ∼3% | Evonik Goldschmidt |
| Texapon N 70 | Natriumlaurylethersulfat | ∼70% | Cognis |
| Texapon K 14 S Spezial | Natriummyristylethersulfat | ∼70% | Cognis |
| Ucare Polymer JR 400 | Polyquaternium-10 | ∼90% | Dow Chemical |
| Unispheres UE-507 | Lactose + Cellulose + Cl 77007 + Hydroxypropyl Methylcellulose + Vitamin-E-Acetate | 68,5% + 24,5% + 3 % + 3 % + 1% | Induchem |
| Uvinul MS-40 | Benzophenon-4 | 100% | BASF |

### Vergleichsversuch Duschgel

Folgende Produkte wurden hergestellt und in einem Probandentest miteinander verglichen: alle Konzentrationsangaben in Gew.-%

| **Phase** | **INCI** | **Produkt A** (erfinderisch) | **Produkt B** (Vergleichsprodukt) |
|---|---|---|---|
| A | Wasser VES | ad 100 | ad 100 |
| A | Texapon N70 | 9.00 | 9.00 |
| A | Natriumbenzoat | 0.45 | 0.45 |
| A | Natriumsalicylat | 0.40 | 0.40 |
| A | Zitronensäure* | 0.35 | 0.35 |
| A | Rewoderm LI 520-70 ** | 0.10 | 0.10 |
| A | Glycerin | 3.50 | 3.50 |
| A | Glycerylglucosid | 3.50 | - |
| B | Eumulgin HRE 40 | 0.50 | 0.50 |
| B | Mandelöl | 0.01 | 0.01 |
| B | Cetiol HE | 1.75 | 1.75 |
| B | Parfum | 1.00 | 1.00 |
| C₁ | Merquat 550 | 5.00 | 5.00 |
| C₂ | Tego Betain F 50 | 16.00 | 16.00 |
| C₃ | Opulyn 301 | 1.00 | 1.00 |

| | | | |
|---|---|---|---|
| * Menge variabel zur Einstellung eines pH-Wertes von 4,8 bis 5,3 ** Menge variabel zur Einstellung einer Viskosität im Bereich von 3000 bis 5000 mPas (gemessen mit HAAKE viscotester VT02 mit Rotor 1) | | | |

### Herstellung

Die Inhaltsstoffe der Phase A werden vermischt, bis eine homogene Phase entsteht.

Eumulgin HRE 40 wird bei einer Temperatur von ca. 40°C aufgeschmolzen. Die übrigen Rohstoffe der Phase B werden zum Eumulgin HRE 40 gegeben. Die Phase wird homogen vermischt und zur Phase A gegeben.

Die Zugabe der Inhaltsstoffe der Phase C erfolgt unter Rühren in der angegebenen Reihenfolge.

Beide Duschgele haben einen L/D-Wert von ca. 1,5.

**Ergebnisse**

| **Kriterium** | **Produkt A** besser | kein Unterschied | **Produkt B** besser |
|---|---|---|---|
| Dufteindruck | 2 | 7 | 0 |
| Konsistenz | 4 | 5 | 0 |
| Schaum menge | 5 | 4 | 0 |
| Schaumqualität | 6 | 3 | 0 |
| Hautgefühl beim Waschen | 8 | 1 | 0 |
| Abspülbarkeit | 2 | 7 | 0 |
| Hautgefühl nach der Anwendung | 3 | 6 | 0 |

7 von 9 Probanden beurteilten die Sensorik des erfinderischen Produktes A insgesamt besser als die Sensorik des Vergleichsproduktes B. Die übrigen Probanden konnten keinen Unterschied feststellen.

Es ist deutlich zu erkennen, dass das erfinderische Produkt A bezüglich aller Merkmale dem Vergleichsprodukt deutlich überlegen ist.

### Vergleichsversuch Gesichtsreiniger

Folgende Produkte wurden hergestellt und in einem Probandentest miteinander verglichen: alle Konzentrationsangaben in Gew.-%

| **Phase** | **INCI** | **Produkt A** (erfinderisch) | **Produkt B** (Vergleichsprodukt) |
|---|---|---|---|
| A₁ | Wasser VES | ad 100 | ad 100 |
| A₁ | Texapon K 14 S Spezial | 4.20 | 4.20 |
| A₁ | Glycerylglucosid | 3.50 | - |
| A₁ | Glycerin | 3.50 | 3.50 |
| A₂ | Plantacare 1200 UP | 2.00 | 2.00 |
| A₃ | Tego Betain F 50 | 11.50 | 11.50 |
| A₄ | Carbopol Aqua SF-1 | 7.50 | 7.50 |
| B | Eumulgin HRE 40 | 0.50 | 0.50 |
| B | Parfum | 0.40 | 0.40 |
| B | Rewoderm LI 520-70 | 0.70 | 0.70 |
| B | Methylparaben | 0.35 | 0.35 |
| B | Propylparaben | 0.35 | 0.35 |
| B | Phenoxyethanol | 0.90 | 0.90 |
| C | Ucare Polymer JR 400 | 0.10 | 0.10 |
| C | Extrapon Gurke | 0.01 | 0.01 |
| C | Wasser VES | 4.00 | 4.00 |
| D₁ | Natronlauge 45%ig * | 0.70 | 0.70 |
| D₂ | Cosmospheres WTS-M | 0.30 | 0.30 |

| | | | |
|---|---|---|---|
| * Menge variabel zur Einstellung eines pH-Wertes von 6,2 bis 6,8 | | | |

### Herstellung

Die Inhaltsstoffe der Phase A mit dem Index 1 werden miteinander vermischt, bis eine homogene Phase entsteht. Die übrigens Inhaltsstoffe der Phase A werden in der angegebenen Reihenfolge zugegeben. Carbopol Aqua SF-1 wird vor der Zugabe filtriert. Es wird gerührt, bis eine klare Phase entstanden ist.

Eumulgin HRE 40 wird bei einer Temperatur von ca. 40°C aufgeschmolzen. Die übrigen Rohstoffe der Phase B werden zum Eumulgin HRE 40 gegeben. Die Phase wird homogen vermischt und zur Phase A gegeben.

Ucare Polymer JR 400 wird in das Wasser der Phase C eingestreut. Die Phase C wird unter Rühren auf ca. 70°C erwärmt bis eine klare Lösung entsteht. Phase C wird gekühlt und zur Phase A gegeben.

Die Inhalststoffe der Phase D werden in der angegebenen Reihenfolge zugegeben. Nach Zugabe der Cosmospheres erfolgt eine vorsichtige Durchmischung der Formulierung.

Beide Gesichtsreiniger haben einen L/D-Wert von ca. 20.

**Ergebnisse**

| **Kriterium** | **Produkt A** besser | kein Unterschied | **Produkt B** besser |
|---|---|---|---|
| Dufteindruck | 0 | 8 | 0 |
| Konsistenz | 3 | 5 | 0 |
| Schaum menge | 6 | 1 | 1 |
| Schaumqualität | 8 | 0 | 0 |
| Hautgefühl beim Waschen | 8 | 0 | 0 |
| Abspülbarkeit | 0 | 8 | 0 |
| Hautgefühl nach der Anwendung | 6 | 2 | 0 |

4 von 8 Probanden empfanden das Hautgefühl des erfinderischen Produktes A als weniger schmierig-glitschig als das Hautgefühl des Vergleichsproduktes B. Die übrigen Probanden konnten keinen Unterschied feststellen.

Es ist deutlich zu erkennen, dass das erfinderische Produkt A bezüglich aller Merkmale dem Vergleichsprodukt deutlich überlegen ist.

### Beispielrezepturen

### Shampoozubereitungen

alle Konzentrationsangaben in Gew.-%

| **Phase** | **INCI** | **1.1** | **1.2** | **1.3** |
|---|---|---|---|---|
| A | Wasser VES | ad 100 | ad 100 | ad 100 |
| A | Texapon N 70 | 0.15 | 4.50 | - |
| A | Texapon K 14 S Spezial | 4.00 | 5.00 | 8.00 |
| A | Rewoteric AMC | - | 7.50 | 7.00 |
| A | Rewopol SB CS 50 | 6.00 | - | - |
| A | Plantacare 2000 UP | 9.00 | - | 5.50 |
| A | Atlas G-4280 | 3.20 | - | - |
| A | Glucamate DOE-120 | - | 0.60 | - |
| A | Rewoderm LI 520-70 | 3.00 | - | 2.50 |
| A | Oxetal VD 92 | 3.00 | - | - |
| A | Merquat 550 | - | 1.25 | - |
| A | Milchsäure | - | - | 0.60 |
| A | Purasal S/PF 50 | - | - | 5.00 |
| A | Natriumbenzoat | 0.40 | 0.40 | 0.40 |
| A | Natriumsalicylat | - | 0.20 | 0.20 |
| A | Glycerylglucosid | 0.01 | 0.50 | 1.00 |
| B | Ucare Polymer JR 400 | 0.20 | 0.20 | 0.10 |
| B | Wasser VES | 15.00 | 15.00 | 15.00 |
| C | Eumulgin HRE 40 | 0.50 | 0.20 | 0.15 |
| C | Parfum | 0.60 | 0.80 | 0.90 |
| C | Lamesoft PO 65 | - | - | 5.00 |
| D₁ | Natriumchlorid* | - | 1.50 | - |
| D₂ | Zitronensäure** | 0.15 | 0.55 | 0.40 |

| | | | | |
|---|---|---|---|---|
| * Menge variabel zur Einstellung einer Viskosität im Bereich von 3000 bis 4500 mPas (gemessen mit HAAKE viscotester VT02 mit Rotor 1) ** Menge variabel zur Einstellung eines pH-Wertes von 4,8 bis 5,8 | | | | |

Die Zubereitungen haben einen L/D-Wert von ca. 10.

Die Inhaltsstoffe der Phase A werden miteinander vermischt, bis eine homogene Phase entsteht.

Ucare Polymer JR 400 wird in das Wasser der Phase B eingestreut. Die Phase B wird unter Rühren auf ca. 70°C erwärmt bis eine klare Lösung entsteht. Phase B wird gekühlt und zur Phase A gegeben.

Eumulgin HRE 40 wird bei einer Temperatur von ca. 40°C aufgeschmolzen. Das Parfum wird zum Eumulgin HRE 40 gegeben. Die Phase wird homogen vermischt und zur Phase A gegeben.

Die Zugabe der Inhaltsstoffe der Phase D erfolgt in der angegebenen Reihenfolge. Es wird gerührt bis ein homogenes Shampoo entsteht.

### Duschgelzubereitungen

alle Konzentrationsangaben in Gew.-%

| **Phase** | **INCI** | **2.1** | **2.2** | **2.3** | **2.4** | **2.5** |
|---|---|---|---|---|---|---|
| A | Wasser VES | ad 100 | ad 100 | ad 100 | ad 100 | ad 100 |
| A | Texapon N70 | 9.00 | 7.00 | 4.00 | 6.50 | - |
| A | Texapon K 14 S Spezial | - | - | - | - | 7.00 |
| A | Plantacare 2000 UP | - | - | - | - | 5.00 |
| A | Natriumbenzoat | 0.45 | 0.45 | 0.45 | 0.45 | 0.50 |
| A | Natriumsalicylat | 0.40 | 0.40 | - | 0.40 | 0.20 |
| A | Zitronensäure* | 0.35 | 0.35 | 0.35 | 0.50 | 1.70 |
| A | Rewoderm LI 520-70 ** | 0.10 | 0.10 | 0.10 | | 0.40 |
| A | Glucamate DOE-120 | - | - | - | 0.03 | - |
| A | Glycerin | - | 1.00 | - | 1.00 | - |
| A | Uvinul MS-40 | - | - | 0.50 | - | - |
| A | Ammoniumcitrat | - | - | - | - | 0.12 |
| A | Glycerylglucosid | 0.01 | 3.00 | 1.00 | 0.10 | 5.00 |
| B | Wasser VES | - | - | - | - | 10.00 |
| B | Ucare Polymer JR 400 | - | - | - | - | 0.20 |
| C | Eumulgin HRE 40 | 0.50 | 0.50 | 0.50 | 0.25 | 0.80 |
| C | Mandelöl | 0.01 | - | - | 0.20 | - |
| C | Sonnenblumenöl | - | 0.01 | 0.04 | - | - |
| C | Avocadoöl | - | - | 0.02 | - | - |
| C | Cetiol HE | 1.75 | 2.50 | 2.00 | 1.00 | - |
| C | Polyox WSR-301 | - | - | 0.10 | - | - |
| C | Parfum | 1.00 | 1.00 | 1.00 | 0.20 | 0.50 |
| D₁ | Merquat 550 | 3.00 | 4.00 | 5.00 | - | - |
| D₂ | Tego Betain F 50 | 15.00 | 12.00 | 25.00 | 15.00 | - |
| D₃ | Rewoteric AM C | - | - | - | - | 18.00 |
| D₄ | Opulyn 301 | 1,00 | 1,00 | - | - | - |
| D₅ | Euperlan PK 900 | - | - | 4.00 | - | - |
| D₆ | Euperlan PK 771 | - | - | - | 4.00 | - |

| | | | | | | |
|---|---|---|---|---|---|---|
| * Menge variabel zur Einstellung eines pH-Wertes von 4,8 bis 5,3 ** Menge variabel zur Einstellung einer Viskosität im Bereich von 3000 bis 5000 mPas (gemessen mit HAAKE viscotester VT02 mit Rotor 1) | | | | | | |

Die Inhaltsstoffe der Phase A werden vermischt, bis eine homogene Mischung entsteht.

Ucare Polymer JR 400 wird in das Wasser der Phase B eingestreut. Die Phase B wird unter Rühren auf ca. 70°C erwärmt bis eine klare Lösung entsteht. Phase B wird gekühlt und zur Phase A gegeben.

Eumulgin HRE 40 wird bei einer Temperatur von ca. 40°C aufgeschmolzen. Die übrigen Rohstoffe der Phase C werden zum Eumulgin HRE 40 gegeben. Die Phase wird homogen vermischt und zur Phase A gegeben.

Die Zugabe der Inhaltsstoffe der Phase D erfolgt unter Rühren in der angegebenen Reihenfolge.

Die Zubereitungen 2.1 bis 2.4 haben einen L/D-Wert von ca. 2. Die Zubereitung 2.5 hat einen L/D-Wert von ca. 5.

### Gesichtsreinigerzubereitungen

alle Konzentrationsangaben in Gew.-%

| **Phase** | **INCI** | **3.1** | **3.2** | **3.3** |
|---|---|---|---|---|
| A₁ | Wasser VES | ad 100 | ad 100 | ad 100 |
| A₁ | Texapon K 14 S Spezial | 4.00 | 4.50 | 6.00 |
| A₁ | Glycerin | - | 1.00 | - |
| A₁ | Glycerylglucosid | 1.00 | 0.10 | 0.50 |
| A₂ | Plantacare 1200 UP | 4.00 | 2.00 | 4.00 |
| A₃ | Tego Betain F 50 | 10.00 | 12.00 | 8.00 |
| A₄ | Carbopol Aqua SF-1 | 5.00 | 6.50 | 5.00 |
| B | Eumulgin HRE 40 | 0.50 | 0.50 | 0.50 |
| B | Parfum | 0.40 | 0.40 | 0.40 |
| B | Rewoderm LI 520-70 | 0.70 | 0.70 | 0.70 |
| B | Methylparaben | 0.35 | 0.35 | 0.35 |
| B | Ethylparaben | 0.35 | 0.35 | 0.35 |
| B | Phenoxyethanol | 0.90 | 0.90 | 0.90 |
| B | Magnolienextrakt | - | 0.001 | 0.01 |
| C | Ucare Polymer JR 400 | 0.10 | 0.10 | 0.10 |
| C | Panthenol | 0.10 | - | - |
| C | Trinatrium EDTA | - | 0.10 | 0.20 |
| C | Cl 42090 | - | 0.001 | 0.001 |
| C | Uvinul MS-40 | - | 0.05 | 0.05 |
| C | Wasser VES | 4.00 | 4.00 | 4.00 |
| D₁ | Natronlauge 45%ig * | 0.70 | 0.70 | 0.70 |
| D₂ | Cosmospheres WTS-M | 0.30 | - | - |
| D₃ | Cosmospheres BMM-M | - | - | 0.20 |
| D₄ | Inducos 14/1 | - | - | 0.40 |

| | | | | |
|---|---|---|---|---|
| * Menge variabel zur Einstellung eines pH-Wertes von 6,2 bis 6,8 | | | | |

Die Inhaltsstoffe der Phase A mit dem Index 1 werden miteinander vermischt, bis eine homogene Phase entsteht. Die übrigens Inhaltsstoffe der Phase A werden in der angegebenen Reihenfolge zugegeben. Carbopol Aqua SF-1 wird vor der Zugabe filtriert. Es wird gerührt, bis eine klare Phase entstanden ist.

Eumulgin HRE 40 wird bei einer Temperatur von ca. 40°C aufgeschmolzen. Die übrigen Rohstoffe der Phase B werden zum Eumulgin HRE 40 gegeben. Die Phase wird homogen vermischt und zur Phase A gegeben.

Ucare Polymer JR 400 wird in das Wasser der Phase C eingestreut. Die Phase C wird unter Rühren auf ca. 70°C erwärmt bis eine klare Lösung entsteht. Phase C wird gekühlt und zur Phase A gegeben.

Die Inhaltsstoffe der Phase D werden in der angegebenen Reihenfolge zugegeben. Nach Zugabe der Cosmospheres erfolgt eine vorsichtige Durchmischung der Formulierung.

Die Zubereitungen haben einen L/D-Wert von ca. 20.

| **Phase** | **INCI** | **3.4** | **3.5** | **3.6** |
|---|---|---|---|---|
| A | Wasser VES | ad 100 | ad 100 | ad 100 |
| A | Carbopol 980 | 1.20 | 1.00 | 1.50 |
| A | Keltrol CG-RD | 0.25 | 0.50 | 0.20 |
| B | Texapon N70 | 2.00 | 2.00 | 2.00 |
| B | Plantacare 2000 UP | 0.50 | 0.50 | 0.50 |
| B | Glycerin | 1.00 | - | - |
| B | Glycerylglucosid | 1.00 | 1.00 | 1.00 |
| C | Cetiol HE | 0.50 | 0.50 | 0.50 |
| C | Parfum | 0.10 | 0.10 | 0.15 |
| C | Vitamin-E-Acetat | 0.06 | - | - |
| C | Magnolienextrakt | - | 0.001 | - |
| C | Methylparaben | 0.10 | 0.10 | 0.10 |
| C | Ethylparaben | 0.10 | 0.10 | 0.10 |
| C | Phenoxyethanol | 0.70 | 0.70 | - |
| D | Cl 42090 | 0.0002 | 0.0001 | 0.0002 |
| D | Cl 47005 | - | - | 0.003 |
| D | Cl 16035 | 0.0002 | - | - |
| D | Hostapon CT Paste | 1.80 | 1.80 | 1.80 |
| D | Lotusextrakt | 0.10 | - | - |
| D | Panthenol | 0.10 | - | - |
| D | Uvinul MS-40 | 0.05 | 0.05 | 0.05 |
| D | Wasser VES | 10.00 | 10.00 | 10.00 |
| E1 | Natronlauge 45%ig * | 0.90 | 0.90 | 0.90 |
| E2 | Unispheres UE-507 | - | - | 0.20 |
| E3 | Inducos NO. 13/3 | - | - | 1.20 |

| | | | | |
|---|---|---|---|---|
| * Menge variabel zur Einstellung eines pH-Wertes von 6,2 bis 6,8 | | | | |

Das Carbopol 980 wird in das Wasser der Phase A eingestreut und mit einem Zauberstab homogen dispergiert. Anschließend wird Keltrol CG-RD zugefügt und gerührt, bis eine homogene Phase entsteht.

Die Rohstoffe der Phase B werden miteinander gemischt und gerührt, bis eine homogene Phase entsteht. Phase B wird zu Phase A gegeben.

Die Rohstoffe der Phase C ohne das Parfum werden gemischt und unter Rühren auf 50°C erwärmt, bis eine homogene Phase entsteht. Die Mischung wird abgekühlt und das Parfum wird zugegeben. Phase C wird zur Phase A gegeben.

Das Wasser der Phase D und Hostapon CT Paste werden unter Rühren auf 50°C erwärmt. Es wird gerührt, bis Hostapon CT Paste gelöst ist. Anschließend werden die übrigen Rohstoffe der Phase D zugegeben. Es wird gerührt, bis eine homogene Phase entsteht. Phase D wird zur Phase A gegeben.

Die Inhaltsstoffe der Phase E werden in der angegebenen Reihenfolge zugegeben. Nach Zugabe der Natronlauge erfolgt nur noch eine vorsichtige Durchmischung der Formulierung.

Die Zubereitungen haben einen L/D-Wert von ca. 20.

## Patentansprüche

1. Wirkstoffkombinationen aus
(i) einem oder mehreren milden Tensiden
und
(ii) einem oder mehreren Glucosylglyceriden.

2. Wirkstoffkombinationen nach Anspruch 1, in denen das molare Verhältnis von einem oder mehreren sauren Konservierungsmitteln aus dem Bereich von 100 : 1 bis 1 : 1000, bevorzugt 50 : 1 bis 1 : 500, insbesondere bevorzugt 20 : 1 bis 1 : 200 gewählt wird.

3. Wirkstoffkombinationen nach Anspruch 1, **dadurch gekennzeichnet, dass** das oder die milden Tenside ausgewählt sind aus Alkylpolyglucosiden, bevorzugt Laurylglucosid und Decylglucosid, Betaintensiden, bevorzugt Cocamidopropylbetain oder Cocobetain, Aminosäuretensiden, bevorzugt Natriumlauroylglutamat und Dinatriumcocoylglutamat und ethoxylierten Glyceriden, bevorzugt PEG-7 Glycerylcocoat.

4. Kosmetische Zubereitungen, Wirkstoffkombinationen gemäß einem der Ansprüche 1 bis 3 enthaltend.

5. Kosmetische Zubereitungen gemäß Anspruch 4, in denen das oder die Glucosylglyceride in der Wasser- und/oder Ölphase in Konzentrationen von 0,001 - 40,00 Gew.-%, bevorzugt 0,005 - 15,00 Gew.-%, besonders bevorzugt 0,01 - 12,00 Gew.-%, jeweils bezogen auf das Gesamtgewicht der Zusammensetzung, vorliegen.

6. Kosmetische Zubereitungen gemäß Anspruch 4 oder 5, in denen das oder die milden Tenside ausgewählt sind aus Alkylpolyglucosiden, bevorzugt Laurylglucosid und Decylglucosid, Betaintensiden, bevorzugt Cocamidopropylbetain oder Cocobetain, Aminosäuretensiden, bevorzugt Natriumlauroylglutamat und Dinatriumcocoylglutamat und ethoxylierten Glyceriden, bevorzugt PEG-7 Glycerylcocoat.

7. Verwendung von Zubereitungen nach einem der vorstehenden Ansprüche als waschaktive Zubereitungen.
